Europäisches Patentamt

European Patent Office

Office européen des brevets

(11) Publication number: **0 075 275**
A2

# EUROPEAN PATENT APPLICATION

(21) Application number: 82108531.3

(22) Date of filing: 16.09.82

(51) Int. Cl.³: **A 61 B 1/12**

(30) Priority: 17.09.81 JP 146521/81

(43) Date of publication of application: 30.03.83
Bulletin 83/13

(84) Designated Contracting States: AT BE CH DE FR GB IT
LI NL SE

(71) Applicant: OLYMPUS OPTICAL CO., LTD.,
43-2, 2-chome, Hatagaya Shibuya-ku, Tokyo 151 (JP)

(72) Inventor: Oshima, Yutaka, Nakagawara-ryo 1-60,
Sumiyoshi-cho, Fuchu-shi Tokyo (JP)

(74) Representative: KUHNEN & WACKER
Patentanwaltsbüro, Schneggstrasse 3-5 Postfach 1729,
D-8050 Freising (DE)

(54) Endoscope.

(57) An endoscope includes a gas changeover valve (8) provided in an operating section (3) and constructed by a piston (29) and a cylinder (21), an upstream side gas supply path section (25a) connected to a gas inlet port of the gas changeover valve, for leading gas to the gas changeover valve, a downstream side gas supply path section (25b) connected to a gas outlet port of the gas changeover valve and extending through an insertion section (2), for leading gas from said gas changeover valve, and a flexible flange (30) provided on the piston and capable of engaging the cylinder (21), for releasing gas in the downstream side gas supply section to atmosphere when the gas pressure in the section exceeds a predetermined value.

- 1 -

Endoscope

This invention relates to an endoscope which permits the safe supplying of air and gas to the body cavity interior.

Usually, when examining the body cavity using an endoscope inserted in the body cavity interior, it is necessary to provide a predetermined distance between an examination window provided at the tip of the endoscope and the cavity membrane by feeding air into the cavity. Also, when performing a high frequency treatment by inserting the endoscope into the colon, it is necessary to convert explosive gases such as methane gas (generated from the foodstuff residue) into an inflammable gas to prevent ignition of the gas. When supplying air or gas into the body cavity, if the pressure in the body cavity is excessively increased, pain caused to the patient is also increased and, in an extreme case, rupture of the body cavity is possible.

Japanese Utility Model Publication 26225/1977 discloses a method of preventing an excessive supply of air and gas. According to this method, an exhaust port communicating with the body cavity is provided in an operating section of an endoscope, and a pressure regulating valve is mounted at the exhaust port to hold the pressure in the body cavity to be less than a predetermined pressure. With this construction, however, the aforementioned pressure regulating valve has to be provided in the operating section of the endoscope in addition to an air/liquid supply changeover valve and a gas changeover valve. Therefore, not only the operating section is increased in size but also the maneuvability of the endoscope is reduced. Further, it is necessary to provide an exclusive passage in the operating section such that it communicates with the exhaust port at which the pressure regulating valve is provided. Therefore, the internal construction of the operating section becomes complicated, and extra time and labor are required for the assembly of the endoscope.

An object of the invention is to provide an endoscope, in which a gas changeover valve provided in the operating section is made use of to hold the pressure in the body cavity to be within a predetermined pressure, thus avoiding the size increase of the operating section and undue complication of the internal construction of the operating section.

This invention can be more fully understood from the following detailed description when taken in conjunction with the accompanying drawings, in which:

Figs. 1 to 3 show an embodiment of the endoscope according to the invention, in which: Fig. 1 is a schematic view showing the whole endoscope, Fig. 2 is a sectional view showing a gas changeover valve, and Fig. 3 is an air/liquid supply changeover valve; and

Fig. 4 is a sectional view showing a different embodiment of the gas changeover valve.

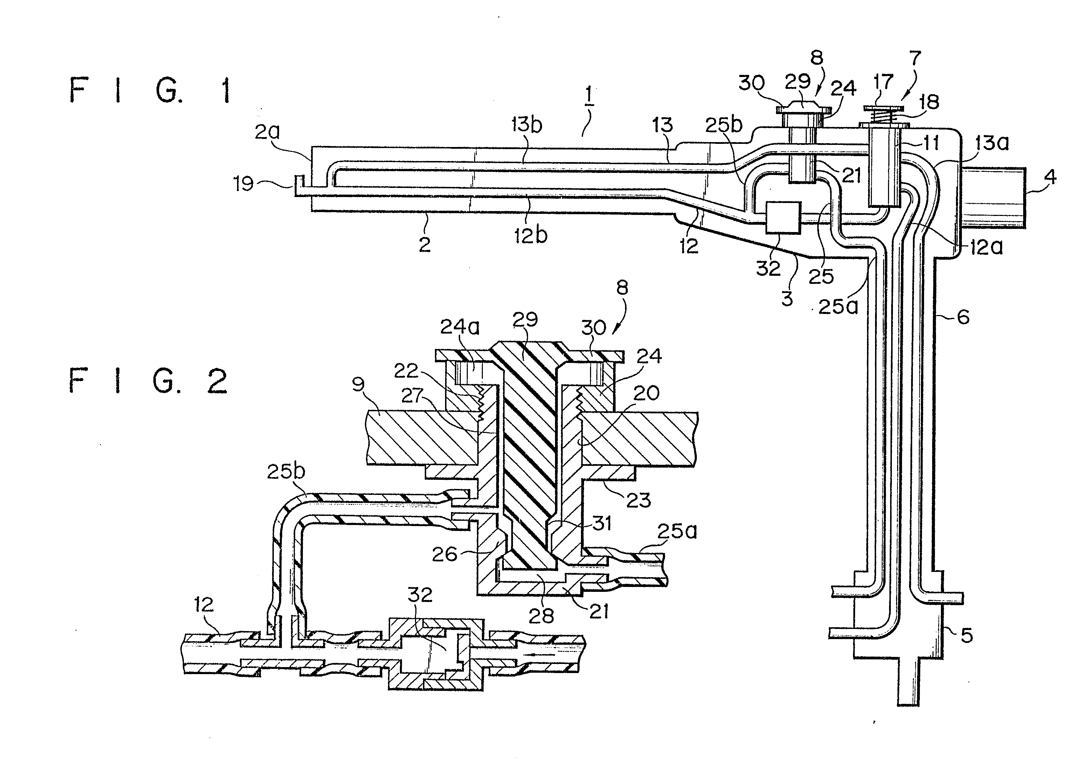

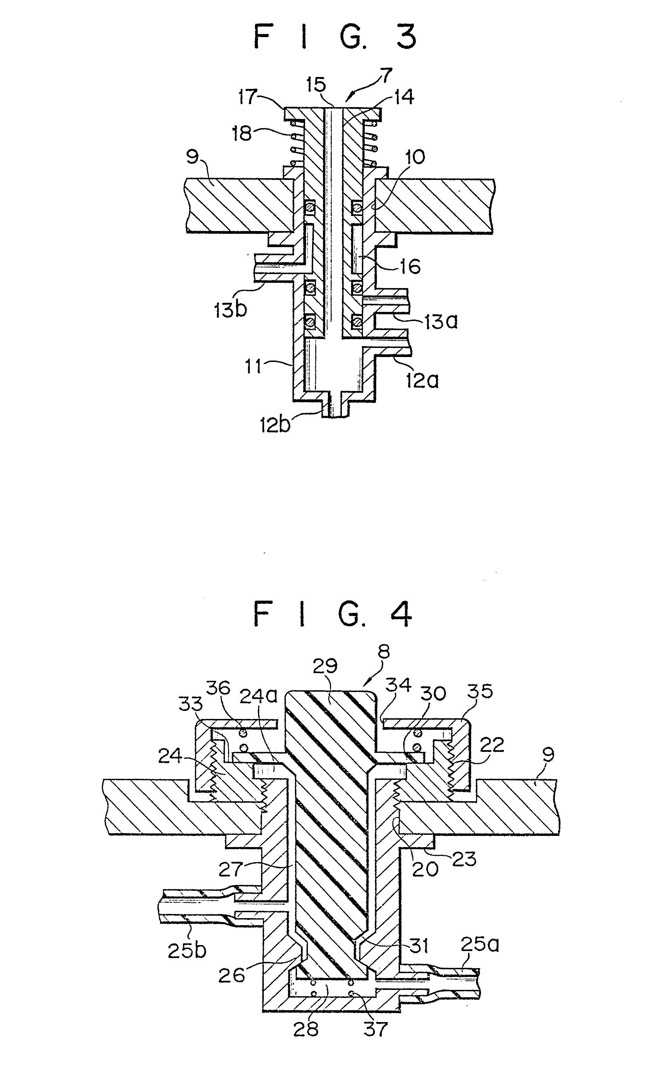

Now, an embodiment of the invention will be described with reference to Figs. 1 to 3. Referring to the Figures, reference numeral 1 designates an endoscope, which comprises an inserting section 2 which is inserted into the body cavity and an operating section 3 for effecting predetermined operations from the outside of the body cavity. The operating section 3 is provided with an eyepiece section 4, and it includes a universal code section 6 having a connector 5 provided at the end. The operating section 3 further includes an air/liquid supply changeover valve 7 and a gas changeover valve 8. The air/liquid supply changeover valve 7 has a construction as shown in Fig. 3. The outer wall 9 of the operating section 3 is formed with a first mounting hole 10. A cylinder 11, which is open at one end and has a bottom at the other end, is securely mounted in the first mounting hole 10 such that its open end extends on the outer side of the operating section 3. The peripheral wall of the cylinder 11 is provided with an inlet port of an upstream portion 12a of an air supply path 12. The peripheral wall is also provided above the air supply path upstream portion inlet port 12a with an inlet port of an upstream portion 13a and an outlet port of a downstream portion 13b of a liquid supply path 13. The bottom of the cylinder is provided with an outlet port of a downstream portion 12b of the air supply path 12. A piston 14 is inserted in the cylinder 11. The piston 14 has a coaxially penetrating leak bore 15, and its outer periphery is formed in an intermediate portion with an annular communication groove 16. The piston 14 has a flange 17 provided at its top projecting from the cylinder 11. A spring 18 is interposed between the flange 17 and the top end of the cylinder 11. When the piston is in the outwardly urged state, the upstream and downstream portions 12a and 12b of the air supply path 12 are communicated with each other while the upstream and downstream portions 13a and 13b of the liquid supply path 13 are not communicated with each other. When the piston 14 is pushed into the cylinder 11 against the restoring force of the spring 18, the upstream and downstream portions 12a and 12b of the air supply path 12 are blocked with respect to each other, while the upstream and downstream portions 13a and 13b of the liquid supply path 13 are communicated with each other by the communicating groove 16 formed in the periphery of the piston 14. The upstream portions 12a and 13a of the air and liquid supply paths 12 and 13 are led through the universal code section 6 to the connector 5 provided at the end of the section. Through the connector 5, the upstream portion 12a of the air supply path 12 is connected to an air pump (not shown), while the upstream portion 13a of the liquid supply path 13 is connected to a liquid container (not shown). The liquid container is adapted to be pressurized by pressurized air from the air supply pump when the air/liquid supply changeover valve 7 is switched to the liquid supply position. The downstream portions 12b and 13b of the air and liquid supply paths 12 and 13 are led through the insertion section 2. These portions are communicated at a position near the end of the intruding section 2, and the unified path is connected to a nozzle 19, which is provided at the end face 2a of the insertion section 2.

The gas changeover valve 8 has a construction as shown in Fig. 2. The casing wall 9 of the operating section 3 is formed with a second mounting hole 2a. A cylinder 21, which is open at one end and has a bottom at the other end, is securely mounted in the second mounting hole 20 such that its open end extends on the outer side of the operating section 3. The outer periphery of an upper portion of the cylinder 21 projecting from the casing wall 9 is formed with a thread 22. The cylinder 21 also has a flange 23 provided at an intermediate portion of its outer periphery. The cylinder 21 is securely mounted in the second mounting hole 20 by screwing a cylindrical rest member 24 onto the thread 22. The rest member 24 has a recess 24a open to the top. The peripheral wall of the cylinder 21 is formed in a lower portion with a gas stream inlet port, through which an upstream portion 25a of a gas supply path 25 is connected to the cylinder. The cylinder peripheral wall is also formed at a position above the gas stream inlet port with a gas stream outlet port, through which a downstream portion 25b of the gas supply path 25 is connected to the cylinder. The inner periphery of the cylinder 21 is formed with an annular ridge 26 having a substantially frustuconical profile extending between the inlet port of the upstream portion 25a and outlet port of the downstream portion 25b of the gas supply path 25. The ridge 26 serves as partitioning means to partition the interior of the cylinder 21 into an upper section (or first section) 27 and a lower section (or second section) 28. The lower section 28 communicates with the upstream portion 25a of the gas supply path 25, while the upper section 27 communicates with the downstream portion 25b of the gas supply path 25 and also with the recess 24a formed in the rest member 24. A piston 29 is inserted in the cylinder 21, and has a diameter smaller than the inner diameter of the cylinder 21. The piston 29 has a concentric valve member 30 in the form of a flange provided at the top of its portion projecting from the cylinder 21. The piston 29 is formed near its lower end with an annular peripheral groove 31 (engaging means), in which the annular ridge 26 is received with a predetermined gap provided between the two. The valve member 30 has elasticity, and its edge portion is in contact with the top of the rest member 24 to hermetically close the recess 24a. The piston 29 is elastically held by the valve member 30. With this elasticity, the lower inclined end face of the groove 31 and the lower inclined end face of the ridge 26 of the cylinder 21 are in contact with each other to hermetically isolate the upper and lower sections 27 and 28 of the cylinder 21 from each other. When the piston 29 is pushed into the cylinder 21 against the spring force of the valve member 30 until its lower end strikes the inner bottom wall of the cylinder 21, the upstream and downstream portions 25a and 25b of the gas supply path 25 are communicated with each other via a gap formed between the lower end surface of the ridge 26 of the cylinder 21 and the lower end surface of the groove 31 of the piston 29. The piston 29 and valve member 30 are made integral with each other by molding of an elastic material such as a synthetic resin or rubber.

The upstream portion 25a of the gas supply path 25 is led through the universal code section 6 to the connector 5 provided at the end thereof, and through the connector it is connected to a gas bomb (not shown) in which an inflammable gas is stored. The downstream portion 25b of the gas supply path 25 is connected to the downstream portion 12b of the air supply path 12 in the operating section 3. A portion of the downstream portion 12b of the air supply path 12 extending between a portion connected to the downstream portion 25b of the gas supply path 25 and the cylinder 11 of the air/liquid supply changeover valve 7, is provided with a check valve 32. The check valve 32 regulates the flow through the downstream portion 12b of the air supply path 12 in the direction as shown by the arrow in Fig. 2.

The operation of the endoscope having the above construction will now be described. When examining the body cavity by inserting the inserting section 2, the air supply pump which is connected to the upstream portion 12a of the air supply path 12 is first driven. When the air supply pump is driven, pressurized air is led to the air/liquid changeover valve 7. If the air/liquid changeover valve 7 is not operated as shown in Fig. 3, the pressurized air flows into the cylinder 11, and after passing through the leak bore 15 of the piston 14, which offers less resistance against flow than that offered by the downstream portion 12b of the air supply path 12, it is dispersed into the atmosphere. When it is desired to supply air into the body cavity, the leak bore 15 of the piston 14 is closed with a finger. When this is done, compressed air, which can no longer escape through the leak bore 15, pushes open the check valve 32 so that it is supplied into the body cavity through the downstream portion 12b of the air supply path 12.

When it is desired to supply liquid, the piston 14 of the air/liquid changeover valve 7 is pushed into the cylinder with the leak bore 15 held closed with a finger. When this is done, the upstream and downstream portions 12a and 12b of the air supply path 12 are isolated from each other by the piston 14 while the upstream and downstream portions 13a and 13b of the liquid supply path 13 are communicated with each other via the communication groove 16 formed in the piston 14. At this time, the liquid container has been pressurized by the pressurized air from the air supply pump. Thus, liquid from the liquid container is forced out to flow through the upstream portion 13a of the liquid supply path 13 and communication groove 16 of the piston 14 into the downstream portion 13b of the path and thence flows out from the nozzle 19 provided at the end face 2a of the intruding section 2.

When it is desired to supply the inflammable gas into the body cavity, the piston 29 of the gas changeover valve 8 is pushed into the cylinder against the elastic force of the valve member 30. As a result, the upper and lower sections 27 and 28 in the gas changeover valve 8, that have been isolated from each other by the contact between the lower end surfaces of the ridge 26 and groove 31, are communicated with each other via the gap formed between these lower end surfaces. Thus, inflammable gas from the gas bomb is caused to flow out through the upstream portion 25a of the gas supply path 25 into the groove 31 in the piston 29 and then into the downstream portion 25b of the path, and issue from the nozzle 19 provided at the end face of the insertion section 2.

In the operation of supplying air or gas as described above, if air or gas is excessively supplied so that the pressure of air or gas in the body cavity is increased beyond a tolerable pressure, this pressure in the body cavity is transmitted through the downstream portion 12b of the air supply path 12 to the downstream portion 25b of the gas supply path 25 so that the pressures in these portions 12b and 25b of paths become equal. This pressure is transmitted from the downstream portion 25b of the gas supply path 25 to the upper section 27 in the gas changeover valve 8, so that the pressure in the recess 24a in the rest member 24 communicating with the upper chamber 27 becomes equal to the aforementioned pressure. This pressure has an effect of pushing out the edge portion of the valve member 30, that has been closing the recess 24a, from the top of the rest member 24, that is, the recess 24a is communicated with the atmosphere. Thus, excessively supplied air or gas in the body cavity is dispersed into the atmosphere through the opened recess 24a until the pressure is reduced to a predetermined level. With the reduction of the pressure to the predetermined pressure, the valve body 30 is brought into contact with the rest member 24 again to hermetically close the recess 24a. Thus, the pressure in the body cavity can be held substantially constant. That is, there is no possibility that the pressure in the body cavity will increase beyond a tolerable pressure level even if air or gas is excessively supplied.

The above embodiment of the invention is by no means limitative, and the gas changeover valve 8 may have a construction as shown in Fig. 4 as well. In this embodiment, the inner periphery of the recess 24a of the rest member 24 is formed with a stepped portion 33. The edge portion of the valve member 30 is rested on top of the stepped portion 33. A cap 35, which is formed at the top with a hole or opening 34 having a sufficiently greater diameter than the diameter of the piston 29, is screwed on the rest member 24. An upper portion of the piston 29 projects through the opening 34. An adjustment spring 36 which is a compression coil spring is interposed between the inner surface of the top wall of the cap 35 and the edge portion of the top of the valve member 30. A retainer spring 37 is interposed between the lower end of the piston 29 and the inner bottom wall of the cylinder 21. The piston 29 is thus spring biased in the projecting direction to hold the inclined lower end surfaces of the ridge 26 and groove 31 in close contact with each other.

With this construction, the hermetical isolation of the upper section 27 from the lower section 28 in the cylinder 21 can be ensured with the restoring force of the retaining spring 37. Also, the recess 24a can be reliably hermetically closed by the valve member 30 because the edge portion of the valve member 30 is urged by the adjustment spring 36. Further, the spring force provided by the adjustment spring 36 urging the valve member 30 can be adjusted by turning the cap 35. By so doing, it is possible to set the tolerable pressure in the body cavity, with which the hermetically closed state of the recess 24a is broken, to a desired pressure.

While in the above embodiment the air/liquid changeover valve has been provided in the operating section, this is by no means limitative; for instance, only the liquid path may be provided in the operating section, and air may be supplied from an air supply source which is provided with an air supply valve disposed on the outside of the endoscope.

As has been described in the foregoing, according to the invention, the gas changeover valve provided on the gas supply path includes a valve member which is opened when the pressure in the gas supply path exceeds a predetermined pressure. Thus, when excessive air or gas is supplied into the body cavity through the gas or air supply path, the valve member is opened to release extra air or gas into the atmosphere, so that there is no possibility of increasing the pressure within the body cavity beyond a tolerable level. Further, since the extra air or gas is allowed to be released through the air or gas supply path, no exclusive discharge paths (as in the prior art) are needed, and so the construction can be simplified. Further, with the valve body provided in the gas changeover valve, instead of providing it separately from the gas changeover valve, considerable size reduction of the operating section can be obtained.

Claims:

1. An endoscope comprising:

an operating section (3);

an insertion section (2) connected to said operating section;

a gas changeover valve (8) provided in said operating section (3) and having a gas inlet port and a gas outlet port;

an upstream side gas supply path section (25a) connected to said gas inlet port of said gas changeover valve, for leading gas to said gas changeover valve; and

a downstream side gas supply path section (25b) connected to said gas outlet port of said gas changeover valve and extending through said insertion section, for leading gas from said gas changeover valve,

characterized by comprising:

a valve body (30) provided in said gas changeover valve (8), for releasing gas in said downstream side gas supply section to atmosphere when the gas pressure in said section exceeds a predetermined value.

2. The endoscope according to claim 1, characterized in that said gas changeover valve (8) includes a cylinder (21) securely mounted in said operating section (3) and formed with said gas inlet port and gas outlet port, and a piston (29) provided in said cylinder (21) and capable of being moved between a first position to communicate said gas inlet port and gas outlet port and a second position to isolate both said ports from each other.

3. The endoscope according to claim 2, characterized by further comprising an air supply path (12) having an intermediate section connected to gas outlet port.

4. The endoscope according to claim 3, characterized by further comprising an air supply valve (7) provided in said operating section (3) and having an inlet port and an outlet port, and in that said air supply path (12) includes an upstream side air supply path section (12a) connected to said inlet port of said air supply valve for leading air into said air supply valve and a downstream side air supply path section (12b) connected between said outlet port of said air supply valve and said downstream side gas supply path section for leading air from said air supply valve to said gas supply path.

5. The endoscope according to claim 4, characterized by further comprising means (32) provided on said downstream side air supply path section (12b) for preventing flow of fluid from said downstream side air supply path section to said air supply valve.

6. The endoscope according to claim 5, characterized in that said means (32) is a check valve.

7. The endoscope according to claim 6, characterized by further comprising a liquid supply path (13) having an end section extending through said insertion section, and in that said air supply valve (7) is provided on an intermediate portion of said liquid supply path (13) for controlling flow of liquid through said liquid supply path.

8. The endoscope according to any one of claims 2 to 7, characterized in that said cylinder (21) includes separating means (26) formed on the intermediate portion of the inner periphery of the cylinder for partitioning the interior of the cylinder into a first section (28) and a second section (27), said gas inlet port being connected to said first section (28), said gas outlet port being connected to said second section (27), and said piston (29) includes engaging means (31) capable of being engaged with said separating means (26) such that said first and second sections are communicated with each other when said piston is in said first position and said first and second sections are isolated from each other when said piston is in said second position.

9. The endoscope according to claim 8, characterized in that said second section (27) includes a gap defined between the inner periphery of said cylinder (21) and the outer periphery of said piston (29).

10. The endoscope according to claim 9, characterized in that said valve body (30) includes a flexible flange provided on said piston (29) and capable of engaging said cylinder (21) to seal said second section.

11. The endoscope according to claim 9, characterized in that said valve body (30) includes a flange provided on said piston (29), a first biasing member (36) for biasing said piston in one direction to have said flange in forced contact with said cylinder so as to seal said second section, and a second biasing member (37) for biasing said piston in the opposite direction with a biasing force less than the biasing force of said first biasing member.

F I G. 1

F I G. 2

# F I G. 3

# F I G. 4